# EUROPÄISCHE PATENTSCHRIFT

(11) **EP 1 654 886 B1**
(45) Veröffentlichungstag und Bekanntmachung des Hinweises auf die Patenterteilung: **30.12.2009**
(21) Anmeldenummer: 04763289.8
(22) Anmeldetag: 16.07.2004
(51) Int. Cl.: H04Q 1/00

(54) **FUNK-BEDIENSYSTEM UND VERFAHREN ZUM BETRIEB EINES FUNKSYSTEMS**
RADIO OPERATING SYSTEM AND METHOD FOR OPERATING A RADIO SYSTEM
SYSTEME PERMETTANT DE FAIRE FONCTIONNER UN SYSTEME RADIO ET PROCEDE PERMETTANT DE FAIRE FONCTIONNER UN SYSTEME RADIO

(30) Priorität: 11.08.2003 DE 10336731
(43) Veröffentlichungstag der Anmeldung: 10.05.2006
(73) Patentinhaber: SIEMENS AKTIENGESELLSCHAFT, 80333 München (DE)
(72) Erfinder: KAGERMEIER, Robert, 90427 Nürnberg (DE); MEDLAR, Donal, 91085 Weisendorf (DE); SIERK, Dietmar, 06120 Halle (DE)
(86) Internationale Anmeldenummer: PCT/EP2004/007954
(87) Internationale Veröffentlichungsnummer: WO 2005/018242

(56) Entgegenhaltungen:
- EP-A- 0 514 244
- EP-A- 1 312 332
- DE-A- 4 300 600
- DE-A- 10 116 870
- GB-A- 2 297 667

## Beschreibung

Die Erfindung betrifft ein Funk-Bediensystem, insbesondere für ein medizintechnisches Gerät, sowie ein Verfahren zum Betrieb eines Funksystems.

Zur Bedienung eines Gerätes, insbesondere eines medizintechnischen Gerätes wie beispielsweise eines Röntgengerätes, ist häufig ein nicht ortsfestes Bedienteil vorgesehen. Ein kabelgebundenes Bedienteil ist beispielsweise aus der EP 0 834 891 A2 bekannt. Hieraus ist auch die Möglichkeit bekannt, ein Bedienteil kabellos, beispielsweise über eine Infrarot-Verbindung, mit einer Zentralstation zu verknüpfen. Eine kabellose Verbindung könnte grundsätzlich auch durch eine Funkverbindung hergestellt werden. Dabei wäre im Gegensatz zu einer Infrarot-Verbindung keine Sichtverbindung zwischen dem Bedienteil und der Zentralstation, d.h. dem anzusteuernden Gerät, erforderlich. Dies hat jedoch den Nachteil, dass eine Bedienperson, die sich mit dem Bedienteil vom anzusteuernden Gerät entfernt, durch unbeabsichtigte Auslösung des Bedienteils Einstellungen des Gerätes verändern könnte. Insbesondere im Fall medizintechnischer Geräte kann ein solcher Vorgang äußerst sicherheitskritisch sein. Funk-Bedienungen werden daher üblicherweise nicht für sicherheitstechnisch kritische Funktionen medizintechnischer Geräte verwendet.

In der EP 1 312 332 A1 sind ein Verfahren und eine Vorrichtung zur Fernbedienung eines Operationstisches beschrieben, wobei von einer Bedienungseinheit drahtlos Befehlsignale an den Operationstisch zum Ansteuern eines Elektroantriebs übertragen werden. Die Übertragung der Befehlsignale zum Operationstisch kann erst dann erfolgen, nachdem die Bedienungseinheit ein Infrarotsignal ausgesendet, dieses von einem Infrarot-Empfänger des Operationstisches empfangen mittels einer Sende- und Empfangseinheit des Operationstisches daraufhin ein Funksignal and die Bedienungseinheit übermittelt hat. Wird die zur Übertragung der Infrarot-Kontrollsignale erfoderlche Sichtverbindung zwischen den Geräten unterbrochen, so werden an der Sende- und Empfangseinheit des Operationstisches eingehende Befehlsignale nur dann verarbeitet werden, wenn der Operationstisch ortsfest im Raum fixiert ist.

In der DE 43 00 600 A1 ist eine fernbedienbare Sicherungseinrichtung für ein Kraftfahrzeug beschrieben, wobei im Kraftfahrzeug zwei getrennte Steuereinrichtungen vorgesehen sind, die durch elektromagnetische Wellen unterschiedlicher Übertragungsfrequenzen betätigt werden.

Der Erfindung liegt die Aufgabe zugrunde, die Anwendungsbereiche von Funk-Bediensystemen, insbesondere für medizintechnische Geräte, zu erweitern.

Diese Aufgabe wird erfindungsgemäß gelöst durch ein Funk-Bediensystem mit den Merkmalen des Anspruchs 1 sowie durch ein Verfahren zum Betrieb eines Funksystems mit den Merkmalen des Anspruchs 6. Die im Folgenden im Zusammenhang mit der Vorrichtung aufgeführten Ausgestaltungen und Vorteile gelten analog auch für das Verfahren und umgekehrt.

Das nach der Erfindung bedienbare Funksystem umfasst eine im Allgemeinen ortsfeste Funk-Basisstation sowie ein im Allgemeinen nicht ortsfestes Bedienteil, zusammenfassend auch als die Teilnehmer des Funksystems bezeichnet. Eine Erweiterung um eine beliebige Anzahl zusätzlicher ortsfester oder transportabler Teilnehmer ist möglich. Ohne Beschränkung der Allgemeinheit wird im Weiteren von einer einzigen Funk-Basisstation und einem einzigen Bedienteil ausgegangen, zwischen welchen eine Funkverbindung aufzubauen ist.

Die Funkverbindung zwischen den Teilnehmern des Funksystems kann in verschiedenen Betriebsmodi aufgebaut werden. Hierzu weist einer der Teilnehmer, vorzugsweise die Bedieneinheit, eine Steuereinrichtung mit einem ersten Schwellwert bezüglich eines Empfangskennwertes auf. Der Empfangskennwert bezieht sich beispielsweise als Empfangsqualitätskennwert auf die Empfangsfeldstärke oder, im Fall einer digitalen Funkverbindung, die Bitfehlerrate der übertragenen Daten. Vorzugsweise werden zur Einstufung der Empfangsqualität sowohl die Empfangsfeldstärke als auch die Bitfehlerrate herangezogen. Eine zur Ermittlung des Empfangskennwertes vorgenommene Messung oder Abschätzung der Distanz zwischen den Teilnehmern des Funksystems ist bevorzugt direkt durch eine Laufzeitmessung und/oder indirekt durch die Auswertung der Empfangsqualität vorgesehen. Vereinfachend werden die Begriffe "Empfangskennwert" und "Empfangsqualitätskennwert" auch für Fälle verwendet, in denen die ermittelten Empfangseigenschaften, insbesondere die Empfangsqualität, durch eine Mehrzahl an Parametern charakterisiert sind.

In Abhängigkeit von der Über- oder Unterschreitung des Schwellwerts durch den Empfangskennwert, insbesondere Empfangsqualitätskennwert, wird ein Standard-Betriebsmodus bzw. ein sicherheitsgerichteter Betriebsmodus des Funksystems aktiviert. Eine Informationsübertragung im Funksystem ist mittels zweier verschiedener Befehlssätze, nämlich einem ersten, nicht sicherheitskritischen Befehlssatz und einem zweiten, sicherheitskritischen Befehlssatz möglich. Solange der Betrieb des Funksystems im Standard-Betriebsmodus erfolgt, sind beide Befehlssätze uneingeschränkt nutzbar. Erfolgt dagegen eine Umschaltung auf den sicherheitsgerichteten Betriebsmodus, d.h. verschlechtert sich die Empfangsqualität und/oder erhöht sich die Distanz zwischen den Teilnehmern über die durch den Schwellwert gegebene Grenze hinaus, so ist nur noch der erste, nicht sicherheitskritische Befehlssatz ohne Einschränkungen nutzbar. Hinsichtlich der Nutzung des zweiten Befehlssatzes werden im sicherheitsgerichteten Betriebsmodus automatisch Einschränkungen vorgenommen.

Im einfachsten Fall wird der zweite Befehlssatz im sicherheitsgerichteten Betriebsmodus vollständig gesperrt. Vorzugsweise bleibt jedoch die Nutzung des zweiten Befehlssatzes im sicherheitsgerichteten Betriebsmodus möglich, sofern eine Bestätigungseingabevorrichtung, beispielsweise Bestätigungstaste, betätigt wird. Hierbei ist nach einer ersten Alternative vorgesehen, dass die Nutzung des zweiten Befehlssatzes nur während der Betätigung der Bestätigungseingabevorrichtung freigegeben wird. Nach einer zweiten Alternative wird durch die Betätigung der Bestätigungseingabevorrichtung im sicherheitsgerichteten Betriebsmodus ein Zeitfenster geöffnet, innerhalb dessen alle Befehlssätze und damit der volle Funktionsumfang des Standard-Betriebsmodus freigegeben sind. Die Bestätigungseingabevorrichtung hat in diesem Fall die Funktion einer Triggertaste. In bevorzugter Weise wird das geöffnete Zeitfenster nach Beendigung der mit einem der Befehlssätze ausgelösten Bedienfunktion nachgetriggert und steht somit in einem weiteren Zeitintervall offen für nachfolgende Bedienanforderungen mit einem beliebigen Befehlssatz. Diese Ausführungsform ist insbesondere bei Ausbildung eines Bedienteils als kabelloser Fußschalter vorteilhaft.

Statt einer Einteilung der Funktionen des Bedienteils nach Sicherheitsgesichtspunkten in zwei Befehlssätze kann - abhängig von der Art des anzusteuernden Gerätes - auch eine feinere Abstufung zweckmäßig sein. Beispielsweise kann vorgesehen sein, für Funktionen, die in jedem Fall aus sicherheitstechnischen Gründen freigegeben sein sollten, insbesondere Not-Abschaltfunktionen, soweit diese durch das Bedienteil auslösbar sind, niedrigere Schwellen bezüglich der erforderlichen Empfangsqualität festzusetzen als für sonstige, für den bestimmungsgemäßen Betrieb des anzusteuernden Gerätes benötigte Bedienfunktionen.

Zur Anzeige des aktiven Betriebsmodus, insbesondere bei aktiviertem sicherheitsgerichteten Betriebsmodus, ist vorzugsweise eine optische Anzeigevorrichtung eines Teilnehmers, insbesondere des Bedienteils, vorgesehen. Durch die automatische Einschaltung der optischen Anzeige, beispielsweise in Form einer blinkenden Leuchtanzeige, bei Umschaltung vom Standard-Betriebsmodus auf den sicherheitsgerichteten Betriebsmodus wird der Benutzer auf die am Ort eines Teilnehmers von diesem gemessene geringer werdende Empfangsqualität und/oder auf die größere werdende Distanz zwischen den Teilnehmern des Funksystems hingewiesen. Eine akustische Warnmeldung wird in bevorzugter Ausführung erst dann ausgegeben, wenn der Benutzer beabsichtigt oder unbeabsichtigt eine Eingabevorrichtung, insbesondere Taste, des Bedienteils betätigt, mit welcher eine dem zweiten, sicherheitskritischen Befehlssatz zugehörige Funktion ausgewählt wird.

Durch die lediglich optische, nicht jedoch akustische Meldung im Fall einer Empfangsqualität unterhalb des Schwellwertes wird der das Bedienteil mit sich tragende Benutzer in für weitere Personen nicht störender Weise darauf aufmerksam gemacht, dass er sich am Rande der Reichweite befindet. Hierbei ist auch berücksichtigt, dass die Reichweite der Funkverbindung durch verschiedenste dämpfende Einflüsse, beispielsweise eine Vielzahl in einem Raum, in welchem sich die Teilnehmer des Funksystems befinden, anwesender Personen, gemindert sein kann. Fällt in einem solchen Fall der Empfangskennwert unter den Schwellwert ab, so ist die optische Meldung als Aufforderung zu verstehen, einen geeigneteren Bedienstandort aufzusuchen.

Ist statt der Empfangsqualität am Ort eines der Teilnehmer des Funksystems die Laufzeit eines zwischen den Teilnehmern übermittelten Signals zumindest hauptsächlich bestimmend für die Festsetzung des Empfangskennwertes, so ist damit eine besonders einfache Möglichkeit gegeben, den Bereich, in welchem eine Funkverbindung zwischen den Teilnehmern uneingeschränkt freigegeben wird, örtlich definiert zu beschränken. Auf diese Weise kann beispielsweise zuverlässig ausgeschlossen werden, dass mit einem funkbasierten Bedienteil, welches zur Bedienung eines in einem Raum ortsfest aufgestellten Gerätes vorgesehen ist, von einem Nachbarraum aus sicherheitskritische Funktionen des Gerätes ausgelöst werden.

Nach einer bevorzugten Weiterbildung ist ein zweiter Schwellwert bezüglich des Empfangskennwertes vorgesehen, bei dessen Unterschreitung eine Abschaltung der Funkverbindung zwischen den Teilnehmern vorgesehen ist. Unabhängig davon, ob die Beendigung der Funkverbindung wegen Unterschreitung des zweiten Schwellwertes oder ohne die Festlegung eines solchen definierten Schwellwertes allein aufgrund der physikalischen Gegebenheiten, insbesondere zu großer Entfernung zwischen den Teilnehmern, erfolgt, ist in bevorzugter Ausgestaltung die Ausgabe einer akustischen Meldung vorgesehen, welche den Benutzer über die Einstellung der Funkverbindung informiert. Damit ist auf einfache Weise vermeidbar, dass ein Benutzer das Bedienteil unbeabsichtigt, beispielsweise in einer Tasche, aus dem Empfangsbereich fortträgt.

Der Vorteil der Erfindung liegt insbesondere darin, dass durch die vollständige oder bedingte Sperrung eines Teils des Funktionsumfangs eines Funk-Bediengerätes bei unter einen festgelegten Schwellwert sinkender Funk-Empfangsqualität und/oder über eine durch den Schwellwert gegebene Maximaldistanz steigender, durch Laufzeitmessung ermittelter Distanz zwischen dem Funk-Bediengerät und dem anzusteuernden Gerät die uneingeschränkte Nutzung des Funk-Bedienteils in gewünschter Weise auf den Nahbereich um das anzusteuernde Gerät, insbesondere medizintechnische Gerät, beschränkt bleibt.

Ein Ausführungsbeispiel der Erfindung wird nachfolgend anhand einer Zeichnung näher erläutert. Hierin veranschaulichen jeweils in schematischer Darstellung:

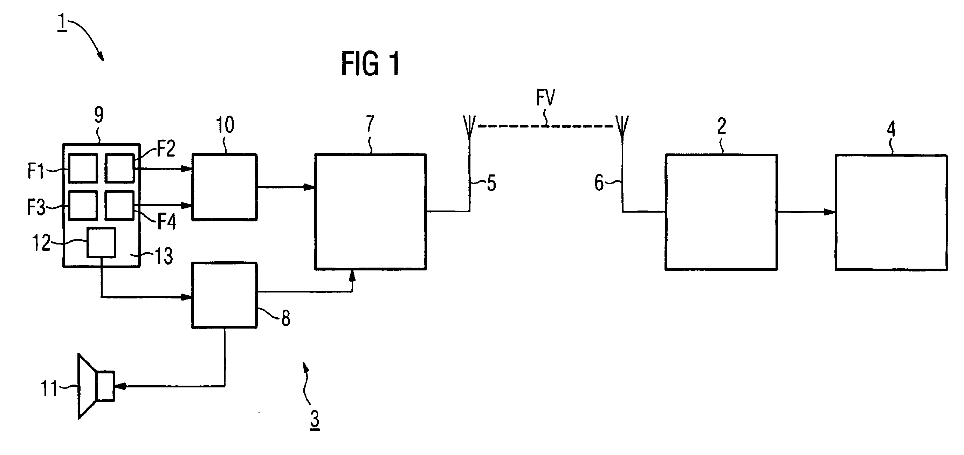

- FIG 1: ein Funk-Bediensystem für ein medizintechnisches Gerät, und
- FIG 2: in einem Flussiagramm verschiedene Betriebszustände des Funk-Bediensystems nach FIG 1.

Einander entsprechende Teile oder Paramter sind in beiden Figuren mit den gleichen Bezugszeichen versehen.

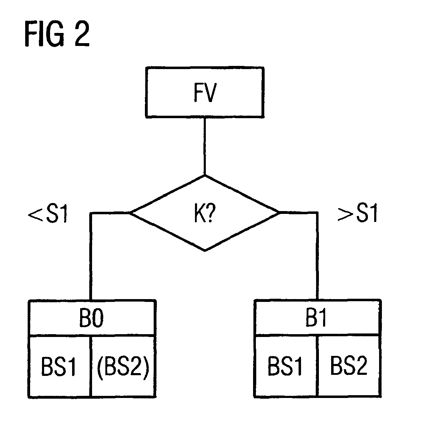

Ein Funk-Bediensystem 1 umfasst eine ortsfeste Funk-Basisstation 2 sowie ein mobiles Bedienteil 3. An die Funk-Basisstation 2 ist ein anzusteuerndes medizintechnisches Gerät 4, beispielsweise ein röntgentechnisches Gerät oder ein Lithotripsiegerät, angeschlossen. Der Funktionsumfang des Gerätes 4 umfasst sicherheitstechnisch relevante Funktionen, beispielsweise die Schaltung von Antrieben oder Strahlungsquellen, sowie sicherheitstechnisch nicht relevante Funktionen, beispielsweise Anzeigefunktionen. Sowohl sicherheitskritische als auch nicht sicherheitskritische Funktionen des Gerätes 4 sind mittels des Bedienteils 3 steuerbar. Eine Funkverbindung zwischen dem Bedienteil 3 und der Funk-Basisstation 2 wird mittels Antennen 5,6 aufgebaut, wobei die maximale Reichweite ca. 5 bis 8 Meter beträgt. In der Regel arbeitet der das Bedienteil 3 betätigende Benutzer unter der Sicherheitsvorgabe, eine Sichtverbindung zum anzusteuernden Gerät 4 und damit im Allgemeinen auch zur Funk-Basisstation 2, welche typischerweise am Gerät 4 installiert ist, einzuhalten. Ist die Funk-Basisstation 2 beispielsweise durch ein von einem Untersuchungtisch herabhängendes Tuch abgedeckt, so beeinträchtigt dies die Funkverbindung, welche gestrichelt angedeutet und mit FV bezeichnet ist, praktisch nicht. Insofern bietet die Funkverbindung zwischen der Funk-Basisstation 2 und dem Bedienteil 3 bedeutsame Vorteile, beispielsweise gegenüber einer Infrarot-Verbindung. Andererseits ist jedoch die Möglichkeit nicht grundsätzlich auszuschließen, dass der Bediener durch unbeabsichtigtes Betätigen des Bedienteils 3 ohne Sichtkontakt zum Gerät 4, beispielsweise in einem Vorraum zum Untersuchungsraum oder OP-Raum, in welchem das Gerät 4 aufgestellt ist, in nicht gewünschter Weise Funktionen des Gerätes 4 auslöst. Dieser Gefahr wird durch den Betrieb des Bedienteils 3 in nachstehend näher beschriebenen Betriebsmodi B0,B1 wirksam begegnet, wobei auch auf das Flussdiagramm nach FIG 2 Bezug genommen wird.

Das Bedienteil 3 weist ein Funkmodul 7 auf, welches eine Information über die mittels der Antenne 5 gemessene Feldstärke sowie über die Bitfehlerrate der empfangenen Daten an eine Steuereinrichtung 8, im folgenden kurz auch als Steuerung bezeichnet, weiterleitet. Zusätzlich oder alternativ wird im Funk-Bediensystem 1 der Abstand zwischen den Antennen 5,6 der Teilnehmer 2,3 durch Laufzeitmessung ermittelt.

Die Steuerung 8 ist weiterhin verbunden mit einer Tastatur 9, einem Tastaturcontroller 10, sowie einem Lautsprecher 11 als akustischer Ausgabevorrichtung. Die Tastatur 9 umfasst im vereinfacht dargestellten Ausführungsbeispiel vier Funktionstasten F1,F2,F3,F4, wobei die Bezeichnungen F1 bis F4 die durch das Bedienteil 3 ansteuerbaren Funktionen des Gerätes 4 kennzeichnen. Hierbei sind die Funktionen F1,F2 einem ersten, nicht sicherheitskritischen Befehlssatz BS1 und die Funktionen F3,F4 einem zweiten sicherheitskritischen Befehlssatz BS2 zugeordnet. Solange der das Bedienteil 3 tragende Benutzer einen geringen, zum regulären Betrieb des Gerätes 4 vorgesehenen Abstand ohne nennenswerte Abschirmungen einhält, sind sämtliche Funktionen F1 bis F4 uneingeschränkt freigegeben: Das Bedienteil 3 befindet sich im Standard-Betriebsmodus B1.

Mit größer werdendem Abstand zwischen dem Bedienteil 3 und der Funk-Basisstation 2 als den Teilnehmern des Funk-Bediensystems 1 und/oder bei zwischen den Teilnehmern 2,3 angeordneten Abschirmungen, beispielsweise in Form einer Wand in einem Gebäude, verschlechtert sich die mittels der Antenne 5 des Bedienteils 3 detektierbare Empfangsqualität des von der Funk-Basisstation 2 ausgehenden Datenstroms. Die am Ort des Bedienteils 3 gegebene Empfangsqualität wird, bevorzugt durch Messung der Feldstärke sowie Ermittlung der Bitfehlerrate, in mindestens einen Empfangsqualitätskennwert K, kurz auch als Empfangskennwert oder Kennwert bezeichnet, umgesetzt, welcher der Steuerung 8 als Eingabegröße dient. Alternativ ist der Empfangskennwert K ein Maß für die Distanz zwischen den Teilnehmern 2,3 des Funksystems, wobei ein geringer werdender Empfangskennwert K einem größer werdenden Abstand zwischen den Teilnehmern 2,3 entspricht. Die Steuerung 8 vergleicht den vom Funkmodul 7 generierten Kennwert K mit einem ersten Schwellwert S1. Die Aufteilung in das Funkmodul 7 einerseits und die Steuerung 8 andererseits ist dabei lediglich symbolisch zu verstehen. Tatsächlich sind beispielsweise mehrere oder alle der Bauteile Funkmodul 7, Steuerung 8 und Tastaturcontroller 10 als einheitliches Bauteil ausgebildet, wobei Steuerungs- und Regelungsfunktionen auch software-technisch realisiert sein können. Ebenso können innerhalb des Bedienteils 3 symbolisiert dargestellte Funktionen alternativ auch in die Funk-Basisstation 2 und/oder das Gerät 4 integriert sein.

Bei Unterschreitung des ersten Schwellwertes S1 durch den Empfangskennwert K, das heißt insbesondere bei geringer werdender Empfangsqualität, wird ein weiterer, sicherheitsgerichteter Betriebsmodus B0 aktiviert. Hierbei sind die dem ersten, nicht sicherheitskritischen Befehlssatz BS1 zugeordneten Funktionen F1,F2 unverändert freigegeben. Dagegen sind die dem zweiten, sicherheitskritischen Befehlssatz BS2 zugeordneten Funktionen F3,F4 nicht durch bloße Betätigung der entsprechenden Tasten der Bedieneinheit 3 nutzbar. Vielmehr ist zur Freischaltung der sicherheitskritischen Funktionen F3,F4 die Betätigung einer Freigabetaste 12 als Bestätigungseingabevorrichtung erforderlich. Hierbei ist nach einer ersten Alternative die Nutzung der Funktionen F3,F4 des sicherheitskritischen Befehlssatz BS2 nur freigegeben, solange die Freigabetaste 12 gedrückt bleibt. Nach einer zweiten Alternative wird durch kurze Betätigung der Freigabetaste 12 der beide Befehlssätze BS1, BS2 umfassende volle Funktionsumfang des Standard-Betriebsmodus B1 für einen Zeitraum von beispielsweise 10 Sekunden freigegeben. Auf die Notwendigkeit zur Betätigung der Freigabetaste 12 wird der Benutzer durch ein vom Lautsprecher 11 ausgegebenes akustisches Warnsignal hingewiesen, sobald er eine der Funktionen F3,F4 im sicherheitsgerichteten Betriebsmodus B0 ohne Freigabe anwählt. Auf diese Weise ist sicher ausgeschlossen, das der Benutzer eine sicherheitskritische Funktion des Gerätes 4 unbeabsichtigt auslöst. Die eingeschränkte, im Ausführungsbeispiel nur nach Betätigung der Freigabetaste 12 mögliche Nutzung des sicherheitskritischen Befehlssatz BS2 im sicherheitsgerichteten Betriebsmodus B0 ist in FIG 2 durch die in Klammern gesetzte Bezeichnung BS2 symbolisiert.

Entfernt sich der Benutzer mit dem Bedienteil 3 aus dem Nahbereich um die Funk-Basisstation 2, in welchem der Standard-Betriebsmodus B0 möglich ist, so erfolgt nicht automatisch eine akustische Warnung. Der in diesem Fall aktivierte sicherheitsgerichtete Betriebsmodus B0 wird jedoch mittels einer Leuchtdiode 13 als Anzeigeeinrichtung in nicht störender Weise angezeigt. Werden ausschließlich die dem nicht sicherheitskritischen Befehlssatz BS1 zugeordneten Funktionen F1,F2 angewählt, so erfolgt keine akustische Meldung. Zugleich ist der Benutzer darüber informiert, dass er sich in einem Bereich mit reduzierter Empfangsfeldstärke und/oder erhöhter Bitfehlerrate befindet. Auf diese Weise ist eine hohe Benutzerfreundlichkeit des Funk-Bediensystems 1 gegeben.

Wird das Bedienteil 3 weiter von der Funk-Basisstation 2 entfernt oder die Empfangsqualität am Ort der Antenne 5 auf andere Weise weiter reduziert, so unterschreitet der Kennwert K einen zweiten in der Steuerung 8 vorgegebenen Schwellwert S2. In diesem Fall werden sämtliche Funktionen F1 bis F4 gesperrt. Eine entsprechende Information des Benutzers erfolgt durch eine über den Lautsprecher 11 ausgegebene akustische Meldung, beispielsweise in Form einer bestimmten Tonfolge oder einer Sprachausgabe. Sofern das Bedienteil 3 zusätzlich zur Tastatur 9 oder mit dieser kombiniert, beispielsweise in Form eines berührungssensitiven Bildschirms, auch eine optische Ausgabevorrichtung aufweist, ist bevorzugt zusätzlich oder alternativ auch die Ausgabe einer optischen Meldung, beispielsweise in Form einer Klartextanzeige, vorgesehen.

## Patentansprüche

1. Funk-Bediensystem mit einer zur Steuerung eines Gerätes vorgesehenen Funk-Basisstation (2) und einem zum Aufbau einer Funkverbindung mit der Funk-Basisstation (2) vorgesehenen Bedienteil (3), wobei
• das Bedienteil (3) eine Steuereinrichtung (8) mit einem ersten Schwellwert (S1) bezüglich eines Empfangskennwertes (K) aufweist, in Abhängigkeit dessen eine Umschaltung zwischen verschiedenen Betriebsmodi (B0,B1) des Bedienteils (3) vorgesehen ist,
• bei Unterschreitung des Schwellwertes (S1) ein sicherheitsgerichteter Betriebsmodus (B0) und bei Überschreitung des Schwellwertes (S1) ein Standard-Betriebsmodus (B1) des Bedienteils (3) vorgesehen ist,
• ein erster, nicht sicherheitskritischer, mittels des Bedienteils (3) aktivierbarer Befehlssatz (BS1) in den verschiedenen Betriebsmodi (B0,B1) gleichermaßen nutzbar ist, und
• ein zweiter, sicherheitskritischer, mittels des Bedienteils (3) aktivierbarer Befehlssatz (BS2) im sicherheitsgerichteten Betriebsmodus (B0) im Vergleich zum Standard-Betriebsmodus (B1) höchstens eingeschränkt nutzbar ist.

2. Funk-Bediensystem nach Anspruch 1, **gekennzeichnet durch** eine Bestätigungseingabevorrichtung (12), **durch** deren Betätigung der sicherheitskritische Befehlssatz (BS2) im sicherheitsgerichteten Betriebsmodus (B0) entsprechend dem Standard-Betriebsmodus (B1) nutzbar ist.

3. Funk-Bediensystem nach Anspruch 1 oder 2, **dadurch gekennzeichnet , dass** das Bedienteil (3) eine zur Anzeige des Betriebsmodus (B0,B1) vorgesehene Anzeigevorrichtung (13) aufweist.

4. Funk-Bediensystem nach einem der Ansprüche 1 bis 3, **dadurch gekennzeichnet, dass** das Bedienteil (3) eine akustische Ausgabevorrichtung (11) aufweist.

5. Funk-Bediensystem nach einem der Ansprüche 1 bis 4, **gekennzeichnet durch** einen zweiten Schwellwert (S2) bezüglich eines Empfangskennwertes (K), bei dessen Unterschreitung eine Abschaltung der Funkverbindung zwischen dem Bedienteil (3) und der Funk-Basisstation (2) vorgesehen ist.

6. Verfahren zum Betrieb eines Funksystems mit mindestens zwei Teilnehmern (2,3), wobei
• die Übertragungsqualität der Funkverbindung zwischen den Teilnehmern (2,3) gemessen wird,
• die Übertragungsqualität anhand eines Empfangskennwertes (K) mit einem Schwellwert (S1) verglichen wird,
• in Abhängigkeit von der Höhe des Empfangskennwertes (K) in Relation zum Schwellwert (S1) verschiedene Betriebsmodi (B0,B1) aktiviert werden, nämlich ein sicherheitsgerichteter Betriebsmodus (B0) bei Unterschreitung und ein Standard-Betriebsmodus (B1) bei Überschreitung des Schwellwertes (S1),
• zum Betrieb der Funkverbindung ein erster, nicht sicherheitskritischer Befehlssatz (BS1) sowie ein zweiter, sicherheitskritischer Befehlssatz (BS2) vorgesehen ist,
• die Nutzung beider Befehlssätze (BS1,BS2) im Standard-Betriebsmodus (B1) uneingeschränkt freigegeben wird,
• im sicherheitsgerichteten Betriebsmodus (B0) lediglich die Nutzung des ersten Befehlssatzes (BS1) uneingeschränkt freigegeben wird, während die Nutzbarkeit des zweiten Befehlsmodus (BS2) eingeschränkt wird.

7. Verfahren nach Anspruch 6, **dadurch gekennzeichnet, dass** der volle Funktionsumfang des Standard-Betriebsmodus (B1) im sicherheitsgerichteten Betriebsmodus (B0) durch Betätigung einer Bestätigungseingabevorrichtung (12) freigegeben wird.

8. Verfahren nach Anspruch 7, **dadurch gekennzeichnet, dass** die Nutzung des vollen Funktionsumfangs des Standard-Betriebsmodus (B1) im sicherheitsgerichteten Betriebsmodus (B0) ausschließlich während der Betätigung der Bestätigungseingabevorrichtung (12) freigegeben wird.

9. Verfahren nach Anspruch 7, **dadurch gekennzeichnet, dass** durch die Betätigung der Bestätigungseingabevorrichtung (12) im sicherheitsgerichteten Betriebsmodus (B0) ein Zeitfenster geöffnet wird, innerhalb dessen der Funktionsumfang des Standard-Betriebsmodus (B1) freigegeben wird.

10. Verfahren nach einem der Ansprüche 6 bis 9, **dadurch gekennzeichnet, dass** bei Umschaltung vom Standard-Betriebsmodus (B1) in den sicherheitsgerichteten Betriebsmodus (B0) eine optische Meldung ausgegeben wird.

11. Verfahren nach einem der Ansprüche 6 bis 10, **dadurch gekennzeichnet, dass** bei Anwahl einer dem sicherheitskritischen Befehlssatz (BS2) zugehörigen Funktion im sicherheitsgerichteten Betriebsmodus (B0) eine akustische Warnmeldung ausgegeben wird.

12. Verfahren nach einem der Ansprüche 6 bis 11, **dadurch gekennzeichnet, dass** bei durch die Übertragungsqualität bedingtem Abbruch der Funkverbindung zwischen den Teilnehmern (2,3) eine akustische Meldung ausgegeben wird.

13. Verfahren nach einem der Ansprüche 6 bis 12, **dadurch gekennzeichnet, dass** der Empfangskennwert (K) eine die Empfangsqualität der Funkverbindung zwischen den Teilnehmern (2,3) betreffende Information beinhaltet.

14. Verfahren nach Anspruch 13, **dadurch gekennzeichnet, dass** der Empfangskennwert (K) eine die Empfangsfeldstärke am Ort eines Teilnehmers (2,3) betreffende Information beinhaltet.

15. Verfahren nach Anspruch 13 oder 14, **dadurch gekennzeichnet , dass** der Empfangskennwert (K) eine die Bitfehlerrate der Funkverbindung zwischen den Teilnehmern (2,3) betreffende Information beinhaltet.

16. Verfahren nach einem der Ansprüche 6 bis 15, **dadurch gekennzeichnet, dass** der Empfangskennwert (K) eine die Distanz zwischen den Teilnehmern (2,3) betreffende Information beinhaltet.

17. Verfahren nach Anspruch 16, **dadurch gekennzeichnet, dass** der Empfangskennwert (K) durch Laufzeitmessung ermittelt wird.

## Claims

1. Radio operating system having a radio base station (2), provided for control of a device, and an operating element (3) for establishment of a radio connection with the radio base station (2), wherein
• the operating element (3) has a controller (8) with a first threshold value (S1) relating to a reception parameter (K), depending on which a switchover between various operating modes (B0, B1) of the operating element (3) is provided,
• on dropping below the threshold value (S1), a safety-oriented operating mode (B0) and, on exceeding the threshold value (S1), a standard operating mode (B1) of the operating element (3) are provided,
• a first non-safety-critical command set (BS1) can be activated by the operating element (3) and equally can be used in the various operating modes (B0, B1), and
• a second safety-critical command set (BS2) can be activated by the operating element (3) and can be used in the safety-oriented operating mode (B0) in a severely limited manner in comparison to the standard operating mode (B1).

2. Radio operating system according to claim 1, **characterised by** a confirmation input device (12), by activation of which the safety-critical command set (B2) can be used in the safety-oriented operating mode (B0) in accordance with the standard operating mode (B1).

3. Radio operating system according to claims 1 or 2, **characterised in that** the operating element (3) has a display device (13) for displaying the operating mode (B0, B1).

4. Radio operating system according to one of claims 1 to 3, **characterised in that** the operating element (3) has an acoustic output device (11).

5. Radio operating system according to one of claims 1 to 4, **characterised by** a second threshold value (S2) relating to a reception parameter (K), the radio connection between the operating element (3) and the radio base station (2) being disconnected if the reception parameter (K) is undershot.

6. Method for operating a radio system having at least two users (2, 3), wherein
• the transmission quality of the radio connection between the users (2, 3) is measured,
• the transmission quality is compared to a threshold value (S1) on the basis of a reception parameter (K),
• depending on the level of the reception parameter (K) in relation to the threshold value (S1) different operating modes (B0, B1) are activated, namely a safety-oriented operating mode (B0) if the threshold value (S1) is undershot and a standard operating mode (B1) if it is exceeded,
• a first, non-safety-critical command set (BS1) and a second, safety-critical command set (B2) are provided for operating the radio connection,
• the use of both command sets (BS1, BS2) is enabled without limitation in the standard operating mode (B1),
• in the safety-oriented operating mode (B0) only the use of the first command set (BS1) is enabled without limitation, while the usability of the second command set (BS2) is limited.

7. Method according to claim 6, **characterised in that** the complete functional scope of the standard operating mode (B1) is enabled in the safety-oriented operating mode (B0) by activating a confirmation input device (12).

8. Method according to claim 7, **characterised in that** the use of the complete functional scope of the standard operating mode (B1) is enabled in the safety-oriented operating mode (B0) solely during activation of the confirmation input device (12).

9. Method according to claim 7, **characterised in that** by activating the activation input device (12) in the safety-oriented operating mode (B0) a time window is opened, within which the functional scope of the standard operating mode (B1) is enabled.

10. Method according to one of claims 6 to 9, **characterised in that** when switching from the standard operating mode (B1) to the safety-oriented operating mode (B0) an optical message is output.

11. Method according to one of claims 6 to 10, **characterised in that** when selecting a function belonging to the safety-critical command set (BS2) in the safety-oriented operating mode (B0) an acoustic warning message is output.

12. Method according to one of claims 6 to 11, **characterised in that** when the radio connection between the users (2, 3) is disconnected as a result of the transmission quality, an acoustic message is output.

13. Method according to one of claims 6 to 12, **characterised in that** the reception parameter (K) contains information concerning the reception quality of the radio connection between the users (2, 3).

14. Method according to claim 13, **characterised in that** the reception parameter (K) contains information concerning the reception field strength at the location of a user (2, 3).

15. Method according to claim 13 or 14, **characterised in that** the reception parameter (K) contains information concerning the bit error rate of the radio connection between the users (2, 3).

16. Method according to one of claims 6 to 15, **characterised in that** the reception parameter contains information concerning the distance between the users (2, 3).

17. Method according to claim 16, **characterised in that** the reception parameter (K) is determined by runtime measurement.

## Revendications

1. Système de commande radio comprenant une station de base radio (2) prévue pour la commande d'un appareil et un organe de commande (3) prévu pour l'établissement d'une liaison radio avec la station de base radio (2),
• l'organe de commande (3) présentant un dispositif de commande (8) comprenant une première valeur seuil (S1) relative à une valeur caractéristique de réception (K), en fonction de laquelle une commutation est prévue entre différents modes de fonctionnement (B0, B1) de l'organe de commande (3),
• un mode de fonctionnement (B0), à sûreté intégrée, de l'organe de commande (3) étant prévue lors du dépassement vers le bas de la valeur seuil (S1) et un mode de fonctionnement standard (B1) de l'organe de commande (3) étant prévu lors du dépassement vers le haut de la valeur seuil (S1) ,
• un premier jeu d'instructions (BS1), non critique quant à la sécurité, activable au moyen de l'organe de commande (3) étant exploitable de la même manière dans les différents modes de fonctionnement (B0, B1), et
• un second jeu d'instructions (BS2), critique quant à la sécurité, activable au moyen de l'organe de commande (3) étant exploitable au maximum de manière restreinte en mode de fonctionnement (B0) à sûreté intégrée, en comparaison avec le mode de fonctionnement standard (B1).

2. Système de commande radio selon la revendication 1, **caractérisé par** un dispositif d'entrée de confirmation (12), par l'activation duquel le jeu d'instructions (BS2) critique quant à la sécurité est exploitable en mode de fonctionnement (B0) à sûreté intégrée, conformément au mode de fonctionnement standard (B1).

3. Système de commande radio selon la revendication 1 ou 2, **caractérisé en ce que** l'organe de commande (3) présente un dispositif d'affichage (13) prévu pour afficher le mode de fonctionnement (B0, B1).

4. Système de commande radio selon l'une quelconque des revendications 1 à 3, **caractérisé en ce que** l'organe de commande (3) présente un dispositif de sortie acoustique (11).

5. Système de commande radio selon l'une quelconque des revendications 1 à 4, **caractérisé par** une seconde valeur seuil (S2) relative à une valeur caractéristique de réception (K), lors du dépassement vers le bas de laquelle une déconnexion de la liaison radio est prévue entre l'organe de commande (3) et la station de base radio (2).

6. Procédé destiné au fonctionnement d'un système radio comprenant au moins deux abonnés (2, 3),
• la qualité de transmission de la liaison radio étant mesurée entre les abonnés (2, 3),
• la qualité de transmission étant comparée à une valeur seuil (S1) à l'aide d'une valeur caractéristique de réception (K),
• différents modes de fonctionnement (B0, B1) étant activés en fonction de la hauteur de la valeur caractéristique de réception (K) en relation avec la valeur seuil (S1) , à savoir un mode de fonctionnement (B0) à sûreté intégrée lors du dépassement vers le bas de la valeur seuil (S1) et un mode de fonctionnement standard (B0) lors du dépassement vers le haut de la valeur seuil (S1),
• un premier jeu d'instructions (BS1) non critique quant à la sécurité, ainsi qu'un second jeu d'instructions (BS2) critique quant à la sécurité étant prévues pour le fonctionnement de la liaison radio,
• l' exploitation des deux jeux d'instructions (BS1, BS2) étant libérée de manière non restreinte en mode de fonctionnement standard (B1)
• seulement l'exploitation du premier jeu d'instructions (BS1) étant libérée de manière non restreinte en mode de fonctionnement (B0) à sûreté intégrée, alors que l'exploitabilité du second mode d'instructions (BS2) est restreinte.

7. Procédé selon la revendication 6, **caractérisé en ce que** le volume total de fonctionnement du mode de fonctionnement standard (B1) en mode de fonctionnement (B0) à sûreté intégrée est libéré par activation d'un dispositif d'entrée de confirmation (12).

8. Procédé selon la revendication 7, **caractérisé en ce que** l'exploitation du volume total de fonctionnement du mode de fonctionnement standard (B1) en mode de fonctionnement (B0) à sûreté intégrée est libérée uniquement pendant l'activation du dispositif d'entrée de confirmation (12).

9. Procédé selon la revendication 7, **caractérisé en ce qu'**en raison de l'activation du dispositif d'entrée de confirmation (12) en mode de fonctionnement (B0) à sûreté intégrée, une fenêtre de temps s'ouvre, pendant laquelle le volume de fonctionnement du mode de fonctionnement standard (B1) est libéré.

10. Procédé selon l'une quelconque des revendications 6 à 9, **caractérisé en ce qu'**un message visuel est sorti lors de la commutation du mode de fonctionnement standard (B1) en mode de fonctionnement (B0) à sûreté intégrée.

11. Procédé selon l'une quelconque des revendications 6 à 10, **caractérisé en ce qu'**un message d'avertissement acoustique est sorti lorsqu'une fonction faisant partie du jeu d'instructions (BS2) critique quant à la sécurité est sélectionnée en mode de fonctionnement (B0) à sûreté intégrée.

12. Procédé selon l'une quelconque des revendications 6 à 11, **caractérisé en ce qu'**un message acoustique est sorti en cas d'interruption de la liaison radio, due à la qualité de transmission, entre les abonnés (2, 3).

13. Procédé selon l'une quelconque des revendications 6 à 12, **caractérisé en ce que** la valeur caractéristique de réception (K) contient une information concernant la qualité de réception de la liaison radio entre les abonnés (2, 3).

14. Procédé selon la revendication 13, **caractérisé en ce que** la valeur caractéristique de réception (K) contient une information concernant l'intensité du champ de réception sur le lieu d'un abonné (2, 3).

15. Procédé selon la revendication 13 ou 14, **caractérisé en ce que** la valeur caractéristique de réception (K) contient une information concernant le taux d'erreurs sur les bits de la liaison radio entre les abonnés (2, 3).

16. Procédé selon l'une quelconque des revendications 6 à 15, **caractérisé en ce que** la valeur caractéristique de réception (K) contient une information concernant la distance entre les abonnés (2, 3).

17. Procédé selon la revendication 16, **caractérisé en ce que** la valeur caractéristique de réception (K) est déterminée par mesure d'un temps de propagation.
